Europäisches Patentamt

European Patent Office

Office européen des brevets

(19)

(11) Veröffentlichungsnummer: **0 392 048**

**A1**

## (12) EUROPÄISCHE PATENTANMELDUNG

(21) Anmeldenummer: **89106520.3**

(22) Anmeldetag: **12.04.89**

(51) Int. Cl.⁵: **A61N 1/365**

(43) Veröffentlichungstag der Anmeldung:
**17.10.90 Patentblatt 90/42**

(84) Benannte Vertragsstaaten:
**DE FR GB IT NL SE**

(71) Anmelder: **Siemens Elema AB**
**Röntgenvägen 2**
**S-171 95 Solna 1(SE)**

(84) **SE**

Anmelder: **Siemens Aktiengesellschaft**
**Wittelsbacherplatz 2**
**D-8000 München 2(DE)**

(84) **DE FR GB IT NL**

(72) Erfinder: **Däne, Niels Thomassen, Dipl.-Ing.**
**Mandolinvägen 43**
**S-175 49 Järfälla(SE)**

(54) **Implantierbares medizinisches Gerät zum Stimulieren einer physiologischen Funktion eines Lebewesens mit einstellbarer Stimulationsintensität und Verfahren zur Einstellung der Stimulationsintensität.**

(57) Die Erfindung betrifft ein medizinisches Gerät zum bedarfsweisen Stimulieren einer physiologischen Funktion mit an die körperliche Aktivität des Lebewesens angepasster Stimulationsintensität, die nach einem Algorithmus in Abhängigkeit von einem der körperlichen Aktivität entsprechenden Signal ermittelt wird. Dabei ist vorgesehen, dass während Phasen spontaner Tätigkeit der physiologischen Funktion deren spontane Intensität gemessen und mit der entsprechenden gemäss dem Algorithmus ermittelten Stimulationsintensität verglichen wird, wobei im Falle von Abweichungen eine Korrektur des Algorithmus erfolgt.

EP 0 392 048 A1

## Implantierbares medizinisches Gerät zum Stimulieren einer physiologischen Funktion eines Lebewesens mit einstellbarer Stimulationsintensität und Verfahren zur Einstellung der Stimulationsintensität

Die Erfindung betrifft ein in den Körper eines Lebewesens implantierbares medizinisches Gerät mit Mitteln zum bedarfsweisen Stimulieren einer physiologischen Funktion des Lebewesens mit einstellbarer Stimulationsintensität, mit einer ein der körperlichen Aktivität des Lebewesens entsprechendes Signal bildenden Sensoreinrichtung, mit Mitteln zum Implementieren eines Algorithmus zur Ermittlung einer an die jeweils vorhandene körperliche Aktivität angepassten Stimulationsintensität in Abhängigkeit von dem Signal der Sensoreinrichtung und mit Stellmitteln, die die jeweils gemäss dem implementierten Algorithmus angepasste Stimulationsintensität einstellen. Die Erfindung betrifft ausserdem ein Verfahren zum Einstellen der Stimulationsintensität eines Gerätes zum bedarfsweisen Stimulieren einer physiologischen Funktion eines Lebewesens mit an die körperliche Aktivität des Lebewesens angepasster Stimulationsintensität. Dabei soll der Begriff Stimulationsintensität umfassend verstanden werden, d.h., dass die Dauer, die Häufigkeit, die Folgefrequenz, die Amplitude usw., mit der die Mittel zum Stimulieren tätig werden, einzeln oder in Kombination als Mass für die Stimulationsintensität verstanden werden sollen.

Mittels derartiger Geräte soll erreicht werden, dass ein Lebewesen, das auf ein Gerät zur Stimulation einer physiologischen Funktion angewiesen ist, ein möglichst normales Leben führen kann. Die jeweilige physiologische Funktion soll also in Abhängigkeit von der körperlichen Aktivität des Lebewesens mit einer Stimulationsintensität stimuliert werden, die möglichst genau derjenigen Intensität entspricht, die vorliegen würde, wenn das Lebewesen nicht auf eine Stimulation der physiologischen Funktion angewiesen wäre.

Ein Gerät der eingangs genannten Art ist aus der EP-A-0 080 348 bekannt. Es handelt sich dabei um einen implantierbaren Herzschrittmacher, in dessen Gehäuse ein piezoelektrischer Drucksensor eingebaut ist, der während körperlicher Aktivitäten des den Herzschrittmacher tragenden Lebewesens durch die Bewegungen der Muskeln und dergleichen entstehende mechanische Schwingungen im Körper des Lebewesens, die sich als Druckwellen ausbreiten, aufnimmt und in ein entsprechendes elektrisches Signal umwandelt. Anhand dieses Signals wird die Stimulationsintensität, d.h. die Stimulationsfrequenz, mit der der Herzschrittmacher das Herz beim Ausbleiben von natürlichen Herzschlägen stimuliert, in Abhängigkeit von der körperlichen Aktivität des Lebewesens nach einem Algorithmus ermittelt und mittels der Stellmittel eingestellt. Dabei geht der Algorithmus von einem durchschnittlichen Patienten, von durchschnittlichen Ankopplungsverhältnissen des Drucksensors an den Körper des Patienten und von durchschnittlichen Fertigungstoleranzen bezüglich des Herzschrittmachers, insbesondere bezüglich derjenigen Komponenten, die an der Bildung des der körperlichen Aktivität entsprechenden Signals beteiligt sind, aus. Dies bedeutet, dass die eingestellte, nach dem Algorithmus ermittelte Stimulationsfrequenz nur in Ausnahmefällen mit derjenigen Herzschlagfrequenz übereinstimmt, mit der das Herz des jeweiligen Patienten bei der vorliegenden körperlichen Aktivität spontan schlagen würde. In der Mehrzahl der Fälle wird die für eine bestimmte körperliche Aktivität eingestellte Stimulationsfrequenz mehr oder weniger stark von der spontanen Herzschlagfrequenz abweichen, die der jeweilige Patient bei der gleichen körperlichen Aktivität aufweisen würde. Dabei kann es durchaus der Fall sein, dass die in Abhängigkeit von der körperlichen Aktivität eines Patienten eingestellte Stimulationsfrequenz im Anschluss an die Implantation des Herzschrittmachers für einige Zeit sehr gut den Bedürfnissen des Patienten entspricht, dann aber, z.B. infolge von über den Drucksensor wachsendem Gewebe, mehr und mehr von der spontanen Herzschlagfrequenz abweicht, mit der das Herz des Patienten spontan schlagen würde.

Der Erfindung liegt die Aufgabe zugrunde, ein Gerät und ein Verfahren der eingangs genannten Art so auszubilden, dass die in Abhängigkeit von der körperlichen Aktivität des Lebewesens eingestellte Stimulationsintensität der spontanen Intensität, die bei der gleichen körperlichen Aktivität vorliegen würde, möglichst exakt entspricht.

Der das Gerät betreffende Teil der Aufgabe wird nach der Erfindung dadurch gelöst, dass eine Detektoreinrichtung zum Detektieren von Phasen spontaner Tätigkeit der physiologischen Funktion, Mittel zum Messen der spontanen Intensität der physiologischen Funktion und Mittel zum Vergleichen vorgesehen sind, die einen Vergleich der in einer Phase spontaner Tätigkeit der physiologischen Funktion gemessenen spontanen Intensität mit der in Abhängigkeit von dem während der Phase spontaner Tätigkeit vorliegenden Signal der Sensoreinrichtung gemäss dem implementierten Algorithmus ermittelten angepassten Stimulationsintensität vornehmen, wobei im Falle einer Abweichung eine selbsttätige Korrektur des implementierten Algorithmus derart erfolgt, dass die gemäss dem Algorithmus ermittelte angepasste Stimulationsintensität wenigstens im wesentlichen der gemessenen spontanen Intensität entspricht. Die Erfindung basiert also auf der Überlegung, dass diejenige spontane Intensität der zu stimulierenden physiologischen Funktion, die sich für eine bestimmte körperliche Aktivität des Patienten während einer Phase spontaner Tätigkeit der physiologischen Funktion einstellt, diejenige Intensität ist, die den Bedürfnissen des Patienten am besten angepasst ist. Wenn die physiologische Funktion stimuliert werden muss, hat dies demzufolge mit einer Stimulationsintensität zu erfolgen, die der für die gleiche körperliche Aktivität während Phasen spontaner Tätigkeit der physiologischen Funktion auftretenden spontanen Intensität entspricht. Dies wird im Falle des erfindungsgemässen Gerätes dadurch erreicht, dass erforderlichenfalls eine Korrektur des Algorithmus in der Weise erfolgt, dass die in Abhängigkeit von der körplichen Aktivität nach dem Algorithmus ermittelte angepasste Stimulationsintensität der in Abhängigkeit von der körperlichen Aktivität auftretenden spontanen Intensität der physiologischen Funktion entspricht. Die physiologische Funktion wird also im Bedarfsfalle immer mit einer Stimulationsintensität stimuliert, die derjenigen spontanen Intensität der physiologischen Funktion weitgehend entspricht, die diese bei der jeweils vorliegenden körperlichen Aktivität des Lebewesens besitzen würde, wenn keine Stimulation erforderlich wäre. Abweichungen, wie sie beim Stand der Technik in aller Regel auftreten, sind praktisch ausgeschlossen. Im Gegenteil ist es so, dass eine ständige Anpassung des Algorithmus an die jeweiligen Gegebenheiten erfolgt. Ändert sich z.B. im Falle des erfindungsgemässen Gerätes dessen Charakteristik aufgrund irgendwelcher Umstände derart, dass einer bestimmten körperlichen Aktivität nach der Änderung der Charakteristik ein anderes Signal entspricht als zuvor, zieht dies eine Korrektur des Algorithmus nach sich, so dass auch weiterhin jeweils mit einer Stimulationsintensität stimuliert wird, die der spontanen Intensität entspricht. Dabei treten allenfalls während der verhältnismässig kurzen Zeiträume, die jeweils zur Korrektur des Algorithmus erforderlich sind, unerwünschte Abweichungen auf.

Der Patentanspruch 2 hat eine besonders vorteilhafte Variante der Erfindung zum Gegenstand. Dadurch, dass die Mittel zum Implementieren einen Funktionsspeicher aufweisen, ist es nämlich sehr leicht möglich, einerseits einen Algorithmus zu implementieren und andererseits den Algorithmus zu korrigieren, da dazu lediglich ein Austausch der in dem Funktionsspeicher gespeicherten Daten erfolgen muss. Ausserdem ist ein sehr leichter Zugriff auf die in dem Funktionsspeicher befindlichen Daten möglich, indem das der körperlichen Aktivität entsprechende Signal digitalisiert wird und die entsprechenden digitalen Daten zur Adressierung des Funktionsspeichers verwendet werden.

Der das Verfahren betreffende Teil der Aufgabe ist durch das Verfahren gemäss Patentanspruch 4 gelöst. Die Wirkungsweise und die Vorteile dieses Verfahrens ergeben sich aus der vorangegangenen Beschreibung des erfindungsgemässen Gerätes.

Eine bevorzugte Ausführungsform des erfindungsgemässen Verfahrens ist in dem Patentanspruch 6 angegeben. Demnach wird vor einer erforderlichen Korrektur des Algorithmus zunächst geprüft, ob die gemessene spontane Intensität innerhalb bestimmter Grenzwerte liegt, um zu verhindern, dass eine Korrektur des Algorithmus in physiologisch nicht sinnvoller Weise erfolgt. Bei den Grenzwerten kann es sich demnach beispielsweise um die physiologisch sinnvolle minimale bzw. maximale Stimulationsintensität handeln.

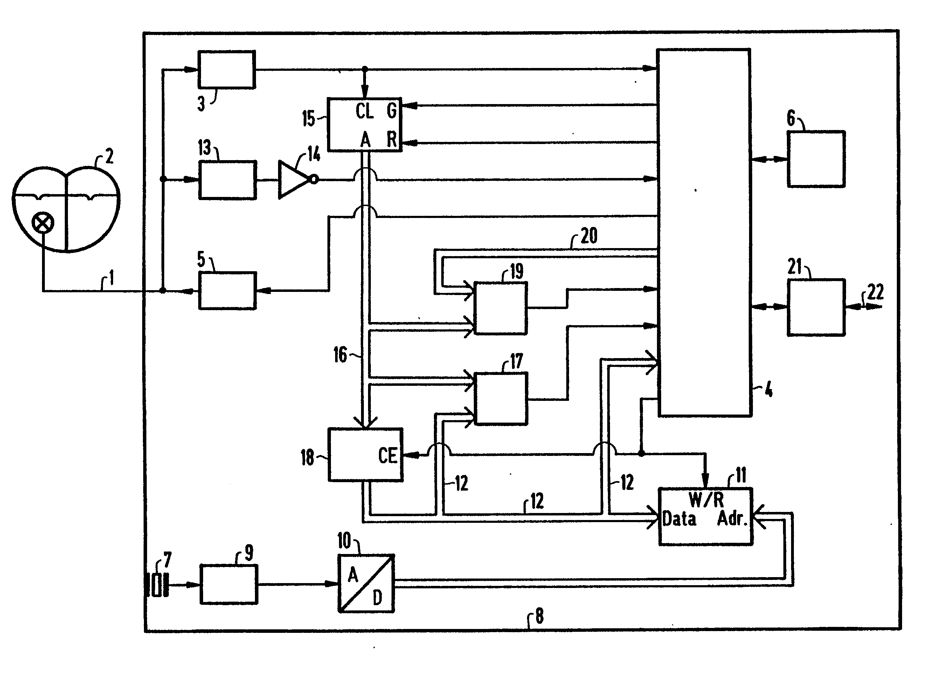

Ein Ausführungsbeispiel der Erfindung wird nachfolgend anhand einer einzigen Figur beschrieben, die das Blockschaltbild eines als Herzschrittmacher ausgebildeten erfindungsgemässen Gerätes zeigt.

Der in der Figur dargestellte Herzschrittmacher ist zur Implantation in den Körper eines Lebewesens vorgesehen. Er arbeitet im VVI-Mode und steht demgemäss über eine in ein Ventrikel des Herzens des Lebewesens eingesetzte Elektrode (1) mit dem Herzen des Lebewesens in Verbindung. Das Herz des Lebewesens ist in der Figur schematisch angedeutet und mit (2) bezeichnet.

Die Elektrode (1) dient dazu, ein der elektrischen Aktivität des Herzens (2) entsprechendes Signal dem Herzschrittmacher zuzuführen. Dort gelangt dieses Signal zu einer Detektoreinrichtung (3), die dazu dient, das Auftreten von spontanen Herzschlägen zu detektieren. Tritt in dem der elektrischen Aktivität des Herzens (2) entsprechenden Signal ein Ereignis mit einer einem natürlichen Herzschlag entsprechenden Mindestamplitude und/oder einer bestimmten, für einen natürlichen Herzschlag typischen Steilheit auf, gibt die Detektoreinrichtung (3) ein das Auftreten eines natürlichen Herzschlages anzeigendes Signal an eine mit (4) bezeichnete Steuerlogik ab.

Der erfindungsgemässe Herzschrittmacher weist ausserdem einen Stimulationsimpuls-Generator (5) auf, der ebenfalls mit der Steuerlogik (4) in Verbindung steht und von dieser zur Abgabe eines elektrischen Stimulationsimpulses zur Stimulation eines Herzschlages aktivierbar ist. Der Ausgang des Stimulationsimpuls-Generators (5) ist ebenfalls mit der Elektrode (1) verbunden, die somit sowohl das der elektrischen Aktivität des Herzens (2) entsprechende Signal dem Herzschrittmacher zuführt als auch die Stimulationsimpulse von dem Herzschrittmacher zu dem Herz (2) leitet. Die Steuerelektronik (4) veranlasst den Stimulationsimpuls-Generator immer dann zur Abgabe eines Stimulationsimpulses, wenn im Anschluss an einen mittels der Detektoreinrichtung (3) detektierten natürlichen Herzschlag oder einen von dem Stimulationsimpuls-Generator (5) abgegebenen Stimulationsimpuls nach Ablauf eines bestimmten Zeitintervalls, des sogenannten Basis-Intervalls, mittels der Detektoreinrichtung (3) kein natürlicher Herzschlag detektiert wurde. Der Herzschrittmacher verhindert also, dass die Herzschlagfrequenz unter eine dem Basis-Intervall entsprechende Frequenz absinkt, da er erforderlichenfalls die Herztätigkeit mit einer Stimulationsfrequenz stimuliert, die dem Basis-Intervall entspricht. Die Dauer des Basisintervalls wird von der Steuerlogik (4) ermittelt, indem diese eine bestimmte Anzahl von Taktimpulsen abzählt, die ihr von einem Taktgenerator (6), z.B. einem Quarz-Oszillator, zugeführt werden.

Im Falle des beschriebenen Herzschrittmachers besitzt die Zeitdauer des Basis-Intervalls keinen festen Wert. Vielmehr ändert sich die Zeitdauer des Basis-Intervalls in Abhängigkeit von der körperlichen Aktivität des Lebewesens derart, dass sich die Stimulationsfrequenz, mit der der Herzschrittmacher erforderlichenfalls stimuliert, ausgehend von einem unteren Grenzwert, z.B. 60 Impulse pro Minute, mit zunehmender körperlicher Aktivität des Lebewesens auf einen oberen Grenzwert, beispielsweise 150 Impulse pro Minute, erhöht. Den Grenzwerten entsprechende Daten sind als Stimulationsimpulse bzw. Herzschläge pro Minute in der Steuerlogik (4) speicherbar. Der Herzschrittmacher simuliert also die Abhängigkeit der spontanen Herzschlagfrequenz von der körperlichen Aktivität durch die Einstellung einer der körperlichen Aktivität angepassten Stimulationsfrequenz.

Um die der jeweiligen körperlichen Aktivität angepasste Stimulationsfrequenz bzw. die Zeitdauer des dieser entsprechenden Basis-Intervalls ermitteln zu können, ist ein piezoelektrischer Drucksensor (7) vorgesehen, der mit der Wandung eines die Elektronik des Herzschrittmachers umgebenden hermetisch dichten Gehäuses (8) verbunden ist. Während körperlicher Aktivitäten des Patienten entstehen durch die Bewegung der Muskeln und dergleichen mechanische Schwingungen im Körper des Patienten, die sich als Druckwellen in dem Körper des Lebewesens ausbreiten und von dem Drucksensor aufgenommen und in elektrische Signale umgewandelt werden. Diese Signale, deren Amplitude mit zunehmender körperlicher Aktivität ebenfalls zunimmt, gelangen zu einer Signalaufbereitungsschaltung (9), die diese Signale filtert und verstärkt. Das Ausgangssignal der Signalaufbereitungsschaltung (9) ist einem Analog/Digital-Wandler (10) zugeführt. Dessen digitale Ausgangssignale sind den Adresseingängen eines Schreib/Lese-Speichers (RAM) (11) zugeführt, der normalerweise im Lesebetrieb arbeitet, wozu seinem zur Umschaltung von Schreib- auf Lesebetrieb und umgekehrt dienenden Eingang W/R von der Steuerlogik (4) ein entsprechendes Signal zugeführt wird. Die Daten-Ein/Ausgänge des RAM (11) sind an einen Datenbus (12) angeschlossen, über den sie u.a. mit der Steuerlogik (4) verbunden sind. Das RAM (11) arbeitet als Funktionsspeicher. Für bestimmte Werte der digitalen Ausgangsdaten des Anaolg/Digital-Wandlers (10), die jeweils einer bestimmten körperlichen Aktivität des Patienten entsprechen, sind in dem RAM (11) Daten bezüglich zugehöriger Werte der Stimulationsfrequenz, im Falle des Ausführungsbeispiels als Stimulationsimpulse bzw. Herzschläge pro Minute und damit der Dauer des Basis-Intervalls gespeichert. In dem RAM (11) ist also sozusagen ein Algorithmus zur Ermittlung einer an die jeweils vorhandene körperliche Aktivität des Patienten angepassten Stimulationsfrequenz in Form einer Funktionstabelle gespeichert.

Die den jeweils vorhandenen Ausgangsdaten des Analog/Digital-Wandlers (10) entsprechenden in dem RAM (11) gespeicherten Daten sind der Steuerlogik (4) zugeführt, die aus diesen Daten die einer an die jeweilige körperliche Aktivität des Patienten angepassten Dauer Basis-Intervalls entsprechende Anzahl von Taktimpulsen des Taktgenerators (6) errechnete und diese Anzahl von Taktimpulsen der Ermittlung des Basis-Intervalls zugrundelegt. Es wird somit deutlich, dass sich die Stimulationsfrequenz bzw. die Zeitdauer des jeweils eingestellten Basis-Intervalls unter Anpassung an die körperliche Aktivität des Lebewesens gemäss den in den RAM (11) gespeicherten Daten ändert.

Um zu erreichen, dass die für eine bestimmte körperliche Aktivität des Patienten eingestellte Stimulationsfrequenz möglichst genau derjenigen Herzschlagfrequenz entspricht, mit der das Herz des Patienten bei der gleichen körperlichen Aktivität spontan schlagen würde, ist im Falle des erfindungsgemässen Herzschrittmachers vorgesehen, dass die spontane Herzschlagfrequenz während solcher Phasen spontaner Herztätigkeit gemessen wird, in denen das Herz dem Sinusrhythmus folgt. Die während einer solchen Phase gemessene spontane Herzschlagfrequenz wird mit der in Abhängigkeit von dem während der jeweiligen Phase vorhandenen körperlichen Aktivität nach dem Algorithmus ermittelten Stimulationsfrequenz verglichen. Im Falle einer Abweichung wird der in dem RAM (11) gespeicherte Algorithmus selbsttätig dahingehend korrigiert wird, dass die gemäss dem Algorithmus ermittelte Stimulationsfrequenz wenigstens im wesentlichen der gemessenen spontanen Herzschlagfrequenz entspricht.

Zu diesem Zweck ist das der elektrischen Aktivität des Herzens entsprechende Signal nicht nur der Detektoreinrichtung (3) sondern ausserdem einem Arrhythmie-Detektor (13) zugeführt, der dann ein Ausgangssignal liefert, wenn eine Arrhythmie vorliegt. Ein solcher Arrhythmie-Detektor ist beispielsweise in der US-PS 3,861,387 beschrieben. Das Ausgangssignal des Arrhythmie-Detektors (13) ist über einen Inverter (14) der Steuerlogik (4) zugeführt, die somit immer dann ein Signal erhält, wenn das Herz dem Sinusrhythmus folgend spontan schlägt. Ausserdem ist ein Zähler (15) vorhanden, der dazu dient, während Phasen spontaner Herztätigkeit nach dem Sinusrhythmus die spontane Herzschlagfrequenz zu ermitteln. Zu diesem Zweck ist der mit C1 bezeichnete Takteingang des Zählers (15) mit dem Ausgang der Detektoreinrichtung (3) verbunden. Wird eine Phase spontaner Herztätigkeit nach dem Sinusrhythmus detektiert, führt die Steuerlogik (4) dem GATE-Eingang G des Zählers (15) für eine definierte Zeitdauer die Steuerlogik (4) durch Abzählen einer entsprechenden Anzahl von Taktimpulsen des Taktgenerators (6) ermittelt, ein Signal zu, das bewirkt, dass die während dieser Zeitdauer auftretenden spontanen Herzschläge gezählt werden. Entsprechende Daten stehen dann am Ende des Zeitintervalls an dem mit A bezeichneten Ausgang des Zählers (15) zur Verfügung, wobei diese Daten die spontane Herzschlagfrequenz in Herzschlägen pro Minute angeben, da das GATE des Zählers (15) im Falle des Ausführungsbeispiels für eine Minute geöffnet wird.

Die Ausgangsdaten des Zählers (15) gelangen über eine Datenleitung (16) zu dem einen Eingang eines digitalen Vergleichers (17), dessen anderem Eingang über den Datenbus (12) diejenigen Daten zugeführt sind, die derjenigen Stimulationsfrequenz entsprechen, die für die der momentanen körperlichen Aktivität des Lebewesens des Patienten entsprechenden digitalen Ausgangsdaten des Analog/Digital-Wandlers (10) in dem RAM (11) gespeichert ist und demzufolge eingestellt würde, wenn eine Stimulation des Herzens (2) erforderlich wäre. Ergibt der Vergleich am Ende des beschriebenen Zählvorgangs eine Abweichung der gemessenen spontanen Herzschlagfrequenz von der für die momentane körperliche Aktivität des Patienten in dem RAM (11) gespeicherten Stimulationsfrequenz, erkennt die Steuerlogik (4) dies an dem entsprechenden Ausgangssignal des Vergleichers (17). Die Steuerlogik (4) schaltet daraufhin das RAM (11) auf Schreibbetrieb um und über einen Tri-State Input/Output-Buffer (18) geeigneter Kanalzahl, dessen mit CE bezeichneter Steuereingang mit dem W/R-Eingang des RAM (11) verbunden ist, die Datenleitung (16) an den Datenbus (12) an. Dies führt dazu, dass die der mittels des Zählers (15) gemessenen spontanen Herzschlagfrequenz entsprechenden Daten anstelle der zuvor vorhandenen Daten in das RAM (11) geschrieben werden. Nach Abschluss des Schreibvorganges schaltet die Steuerlogik (4) das RAM (11) wieder auf Lesebetrieb um, trennt die Datenleitung (16) mittels des Schaltkreises (18) von dem Datenbus (12) und stellt den Zähler (15) durch Zufuhr eines Impulses zu dessen RESET-Eingang R zurück.

Sofern das Ausgangssignal des Inverters (14) weiterhin das Vorhandensein einer Phase spontaner Herztätigkeit nach dem Sinusrhythmus anzeigt, setzt die Steuerlogik (4) den zuvor beschriebenen Vorgang wieder in Gang, in dem sie dem GATE-Eingang G des Zählers (15) ein entsprechendes Signal zuführt.

Es wird somit deutlich, dass die in dem RAM (11) gespeicherten Daten einen Algorithmus zur Ermittlung einer an die körperliche Aktivität des Lebewesens angepassten Stimulationsfrequenz darstellen, der ständig an die jeweiligen Bedingungen angepasst wird. Die nach dem in dem RAM (11) gespeicherten Algorithmus ermittelte Stimulationsfrequenz entspricht also weitestgehend derjenigen Herzschlagfrequenz, mit der das Herz (2) spontan schlagen würde.

Ergibt der Vergleich, dass die gemessene spontane Herzschlagfrequenz mit der für die der momentanen körperlichen Aktivität des Patienten entsprechenden digitalen Ausgangsdaten des Analog/Digital-Wandlers (10) in dem RAM (11) gespeicherten Stimulationsfrequenz übereinstimmen, stellt die Steuerlogik (4) den Zähler (15) durch Zufuhr eines entsprechenden Impulses zu dessen RESET-Eingang R zurück. Ausserdem setzt die Steuerlogik (4) einen neuen Zählvorgang in Gang, indem sie dem GATE-Eingang G des Zählers (15) ein entsprechendes Signal zuführt. Eine Umschaltung des RAM (11) auf Schreibbetrieb unterbleibt.

Endet während eines der beschriebenen, zur Bestimmung der spontanen Herzschlagfrequenz dienenden Zählvorgänge eine Phase spontaner Herztätigkeit nach dem Sinusrhythmus, so wird dieser Zählvorgang abgebrochen, indem die Steuerlogik (4) den GATE-Eingang G des Zählers (15) sperrt und den Zähler (15) durch Zufuhr eines entsprechenden Impulses zu dessen RESET-Eingang R zurückstellt.

Im Falle des beschriebenen Ausführungsbeispiels ist ein weiterer digitaler Vergleicher (19) vorgesehen, dessen einer Eingang mit der Datenleitung (16) verbunden ist, so dass ihm die der jeweils gemessenen spontanen Herzschlagfrequenz entsprechenden Daten zugeführt sind. Dem anderen Eingang des Vergleichers (19) sind von der Steuerlogik (4) über eine Datenleitung (20) diejenigen Daten zuführbar, die dem oberen und dem unteren Grenzwert entsprechen, zwischen denen die Stimulationsintensität einstellbar ist. Bevor die Steuerlogik (4) in der zuvor beschriebenen Weise die Speicherung einer gemessenen spontanen Herzschlagfrequenz in dem RAM (11) veranlasst, wird durch zwei aufeinanderfolgende Vergleichsvorgänge mittels des Vergleichers (19) geprüft, ob die gemessene spontane Herzschlagfrequenz innerhalb der durch den oberen und den unteren Grenzwert der Stimulationsfrequenz gesetzten Grenzen liegt. Dabei erfolgt die Speicherung einer gemessenen spontanen Herzschlagfrequenz nur dann, wenn die Steuerlogik (4) von dem Vergleicher (19) Ausgangssignale erhält, die anzeigen, dass die gemessene Herzschlagfrequenz wenigstens gleich dem unteren und höchstens gleich dem oberen Grenzwert der Stimulationsfrequenz ist. Ist dies nicht der Fall, unterbleibt die Speicherung, so dass sichergestellt ist, dass keine Daten in das RAM (11) gelangen können, die physiologisch nicht sinnvoll bzw. nicht zulässig sind. Stattdessen wird ein neuer Zählvorgang in Gang gesetzt, indem die Steuerlogik zunächst den Zähler (15) durch Zufuhr eines entsprechenden Impulses zu dessen RESET-Eingang R zurückstellt und dann dem GATE-Eingang G des Zählers ein einen neuen Zählvorgang einleitendes Signal zuführt.

An die Steuerlogik (4) ist ein Telemetrieschaltkreis (21) angeschlossen, so dass der Herzschrittmacher in der Lage ist, mit einem nicht dargestellten externen Gerät, einem sogenannten Programmer, bidirektional Daten auszutauschen. Dies ist durch den Doppelpfeil (22) angedeutet. Es besteht somit die Möglichkeit, den Herzschrittmacher zu programmieren. Zum Beispiel können mittels des Programmers über den Telemetrieschaltkreis (21) der obere und der untere Grenzwert für die Stimulationsfrequenz eingegeben werden.

Sofern anders als im Falle des beschriebenen Herzschrittmachers die von dem Zähler (15), dem RAM (11) und der Steuerlogik (4) gelieferten Daten, die sich im Falle des Ausführungsbeispiels jeweils auf Stimulationsimpulse bzw. Herzschläge pro Minute beziehen, nicht miteinander kompatibel sind, können diese nicht unmittelbar den Vergleichern (17) und (19) zugeführt werden. Vielmehr muss zunächst die Kompatibilität der genannten Daten, z.B. durch einen von der Steuerlogik (4) ausgeführten Rechenvorgang, hergestellt werden.

Obwohl die Erfindung anhand eines Herzschrittmachers beschrieben wurde, kann sie auch bei anderen Geräten zur Stimulation einer physiologischen Funktion Anwendung finden. In diesem Falle, aber auch bei Herzschrittmachern, können die für die Erfindung wesentlichen Funktionen auch abweichend von dem zuvor beschriebenen Ausführungsbeispiel realisiert werden. Insbesondere können abweichende Sensoreinrichtungen Verwendung finden. Ausserdem müssen die Mittel zum Implementieren des Algorithmus zur Ermittlung der an die jeweilige Körperaktivität des Patienten angepassten Stimulationsintensität nicht zwangsläufig einen Schreib/Lese-Speicher (11) aufweisen.

Bezugszeichenliste

1 Elektrode
2 Herz
3 Detektoreinrichtung
4 Steuerlogik
5 Stimulationsimpuls-Generator
6 Taktgenerator
7 Drucksensor
8 Gehäuse
9 Signalaufbereitungsschaltung
10 Analog/Digital-Wandler
11 Schreib/Lese-Speicher (RAM)
12 Datenbus
13 Arrhythmie-Detektor
14 Inverter
15 Zähler
16 Datenleitung
17 Vergleicher
18 Schaltkreis
19 Vergleicher
20 Datenleitung
21 Telemetrieschaltkreis
22 Doppelpfeil

**Ansprüche**

1. In den Körper eines Lebewesens implantierbares medizinisches Gerät mit Mitteln (5) zum bedarfsweisen Stimulieren einer physiologischen Funktion des Lebewesens mit einstellbarer Stimulationsintensität, mit einer ein der körperlichen Aktivität des Lebewesens entsprechendes Signal bildenden Sensoreinrichtung (7,9,10), mit Mitteln (11) zum Implementieren eines Algorithmus zur Ermittlung einer an die jeweils vorhandene körperliche Aktivität angepassten Stimulationsintensität in Abhängigkeit von dem Signal der Sensoreinrichtung (7,9,10) und mit Stellmitteln (4), die die jeweils gemäss dem implementierten Algorithmus ermittelte angepasste Stimulationsintensität einstellen, **dadurch gekennzeichnet,** dass eine Detektoreinrichtung (13,14) zum Detektieren von Phasen spontaner Tätigkeit der physiologischen Funktion, Mittel (15) zum Messen der spontanen Intensität der physiologischen Funktion und Mittel (17) zum Vergleichen vorgesehen sind, die einen Vergleich der in einer Phase spontaner Tätigkeit gemessenen spontanen Intensität mit der in Abhängigkeit von dem während der Phase spontaner Tätigkeit vorliegenden Signal der Sensoreinrichtung (7,9,10) gemäss dem implementierten Algorithmus ermittelten angepassten Stimulationsintensität vornehmen, wobei im Falle einer Abweichung eine selbsttätige Korrektur des implementierten Algorithmus derart erfolgt, dass die gemäss dem Algorithmus ermittelte angepasste Stimulationsintensität wenigstens im wesentlichen der gemessenen spontanen Intensität entspricht.

2. Medizinisches Gerät nach Anspruch 1, **dadurch gekennzeichnet,** dass die Mittel (11) zum Implementieren einen Funktionsspeicher aufweisen, in dem der implementierte Algorithmus in Form einer Funktions tabelle gespeichert ist, die für bestimmte Werte des Signals der Sensoreinrichtung (7,9,10) bestimmte Werte der Stimulationsintensität enthält, und dass die Korrektur des implementierten Algorithmus erforderlichenfalls durch Speicherung des für einen Wert des Signals der Sensoreinrichtung (7,9,10) gemessenen Wertes der spontanen Intensität anstelle des zuvor gespeicherten Wertes der Stimulationsintensität erfolgt.

3. Gerät nach Anpruch 1 oder 2, **dadurch gekennzeichnet,** dass das Gerät als Herzschrittmacher ausgebildet ist, wobei die Mittel (5) zum Stimulieren die Herztätigkeit des Lebewesens stimulieren, die Stellmittel (4) die Stimulationsfrequenz einstellen, die Detektoreinrichtung (13,14) diejenigen Phasen der Herztätigkeit detekiert, in denen das Herz des Lebewesens dem Sinusrhythmus folgt, und die Mittel (15) zum Messen der spontanen Intensität die spontane Herzschlagfrequenz messen.

4. Verfahren zum Einstellen der Stimulationsintensität eines Gerätes zum bedarfsweisen Stimulieren einer physiologischen Funktion eines Lebewesens mit an die körperliche Aktivität des Lebewesens angepasster Stimulationsintensität, wobei die körperliche Aktivität des Lebewesens gemessen wird und in Abhängigkeit von dieser nach einem Algorithmus die angepasste Stimulationsintensität ermittelt wird, **dadurch gekennzeichnet,** dass während Phasen spontaner Tätigkeit der physiologischen Funktion die spontane Intensität der physiologischen Funktion gemessen wird, dass die während einer Phase spontaner Tätigkeit gemessene spontane Intensität mit der in Abhängigkeit von der während der Phase spontaner Tätigkeit gemessenen körperlichen Aktivität gemäss dem Algorithmus ermittelten angepassten Stimulationsintensität verglichen wird, und dass im Falle einer Abweichung der Algorithmus selbsttätig derart korrigiert wird, dass die gemäss dem Algorithmus ermittelte angepasste Stimulationsintensität wenigstens im wesentlichen der gemessenen spontanen Intensität entspricht.

5. Verfahren nach Anspruch 4, **dadurch gekennzeichnet,** dass die Herztätigkeit des Lebewesens stimuliert wird, wobei zur Anpassung der Stimulationsintensität die Stimulationsfrequenz eingestellt wird, und dass die spontane Herzschlagfrequenz während solcher Phasen spontaner Herztätigkeit gemessen wird, in denen das Herz dem Sinusrhythmus folgt.

6. Verfahren nach Anspruch 4 oder 5, **dadurch gekennzeichnet,** dass die gemessene spontane Intensität daraufhin überprüft wird, ob sie innerhalb bestimmter Grenzwerte liegt, und nur wenn dies der Fall ist, der Algorithmus erforderlichenfalls korregiert wird.

89 P 7314 E

Europäisches
Patentamt

**EUROPÄISCHER RECHERCHENBERICHT**

Nummer der Anmeldung

EP 89 10 6520

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 228 985 (BIOTRONIK) <br> * Seite 5, Zeile 31 - Seite 13, Zeile 8 * <br><br> --- | 1-5 | A 61 N 1/365 |
| A | EP-A-0 194 224 (BIOTRONIK) <br> * Seite 11, Zeile 4 - Seite 17, Zeile 25 * <br><br> --- | 1-5 | |
| A | EP-A-0 259 658 (INTERMEDICS) <br> * Spalte 9, Zeile 28 - Spalte 10, Zeile 25 * <br><br> --- | 1-5 | |
| A | EP-A-0 256 617 (INTERMEDICS) <br> * Seite 8, Zeile 27 - Seite 12, Zeile 28 * <br><br> ------ | 1-5 | |

**RECHERCHIERTE SACHGEBIETE (Int. Cl.5)**

A 61 N

Der vorliegende Recherchenbericht wurde für alle Patentansprüche erstellt

| Recherchenort | Abschlußdatum der Recherche | Prüfer |
|---|---|---|
| DEN HAAG | 19-12-1989 | LEMERCIER D.L.L. |